# EUROPEAN PATENT APPLICATION

(11) **EP 2 233 192 A1**
(43) Date of publication of application: **29.09.2010**
(21) Application number: 08856295.4
(22) Date of filing: 05.12.2008
(51) Int. Cl.: B01D 43/00, B01J 8/16, A61M 1/34

(54) **MICRO-DEVICE AND METHOD FOR NON-INVASIVE AND SELECTIVE SEPARATION AND EXTRACTION OF PARTICLES IN POLYDISPERSED SUSPENSIONS, PRODUCTION METHOD, AND THE APPLICATIONS THEREOF**

(30) Priority: 05.12.2007 ES 200703248
(71) Applicant: Consejo Superior De Investigaciones Científicas, 28006 Madrid (ES); Ikerlan, 20500 Arrasate-Mondragon (ES); Universidad Miguel Hernández, 03202 Elche (ES); Hospital General Universitario De Elche, 03203 Elche (ES)
(72) Inventor: GONZÁLEZ GOMEZ, Maria Iciar, E-28006 Madrid (ES); GÓMEZ ALVAREZ-ARENAS, Tomas, E-28006 Madrid (ES); FERNANDEZ LEDESMA, Luis José, E-20500 Arrasate-Mondragon (ES); CARRATO MENA, Alfredo, E-03202 Elche (ES); SOTO MARTINEZ, José Luis, E-03203 Elche (ES); BERGANZO RUIZ, Javier, E-20500 Arrasate - Mondragon (ES)
(74) Representative: Pons Ariño, Angel
(86) International application number: PCT/ES2008/070230
(87) International publication number: WO 2009/071733

(57) **Abstract**

The invention relates to a micro-device for non-invasive and selective separation and extraction of particles in polydispersed suspensions by means of the strategic use of ultrasounds, laminar flow and standing wave effects in a channel produced in a chip by means of microtechnology. Said device is a resonant multi-layer system comprising a modified "lambda quarter wavelength" treatment channel, which enables the particles to be channelled and separated in a flow inside the substrate channel without touching the walls of the device, in order to avoid problems of adherence. Said micro-device can be used in the field of biomedicine and/or biotechnology for the separation and concentration of cells, preferably human cells, that can be used for investigative and medical methods for diagnosis and treatment.

## Description

### FIELD OF THE ART

The present invention relates to a device that combines microtechnology and ultrasonic waves as a non-invasive method for the selective separation and extraction of particles in polydispersed suspensions, containing microelements having different physical characteristics (size, density or compressibility) for any concentration level, being mainly applicable to the field of biomedicine and biotechnology.

### STATE OF THE ART

In the last decade, different techniques have been proposed for manipulating or separating suspended particulate matter in different fields of technological application, particularly biotechnology and medicine. At present, the separation of particles is of particular interest to medicine in applications related to blood donations, dialysis processes and laboratory analyses, in addition to the recycling and/or washing of blood after surgical operations.

The application of standing acoustic waves to suspensions produces the effect of transporting particles towards certain equilibrium zones related to the node distribution and maximum acoustic pressure values established by the standing wave generated in the medium. An acoustically induced primary radiation force is exerted on each particle, the magnitude of which varies proportionally to operating frequency. The distance travelled by a particle subjected to this force to reach the nearest acoustic equilibrium position is shorter as the distance between nodes and maximum pressure values becomes shorter. These are defined by the wavelength, which is inversely proportional to acoustic frequency. Therefore, from a theoretical viewpoint, it is simpler to concentrate particles at higher frequencies.

This non-invasive transport mechanism is well known in the field of ultrasound and, during the last decade, has given rise to the development of several attempts to manipulate and/or separate particles. Different techniques for separating particles from a liquid or other fluid using this phenomenon have been proposed. Typically, the fluid circulates through a duct or channel wherein a standing acoustic wave transversal to the length of the channel is established. As a result, the particles move to form concentration bands along the wave equilibrium positions within these ducts.
■ The recent patent WO 2007/044642 A2, "DEVICE AND METHOD FOR COMBINED MICROFLUIDIC-MICROMAGNETIC SEPARATION OF MATERIAL IN CONTINUOUS FLOW" (Ingber Donald, Xia Shannon, Tom Hunt, Peter Westervelt), discloses a continuous flow cell separation device based on the application of magnetic fields. The operating principle is completely different to that of the present invention, as it is not based on the application of mechanical energy but electromagnetic energy and, as opposed to the present invention, is an invasive technology that requires the insertion of inorganic microparticles external to the suspension to be treated. These microparticles witch are susceptible to the magnetic field, become adhered to and drag certain cells of the suspension, magnetically transporting them towards preselected equilibrium zones. Due to being an invasive method, the viability of the separated cells is partially altered for carrying out subsequent studies, once extracted from the medium. These two characteristics of the device make the patent very different from the device of the present invention, although both coincide in the use of a dual fluidized bed under laminar flow regime with one interface.
■ The paper by J. Statis. Mech.: "THEORY AND EXPERIMENTS, 2006, "CONTINUOUS PARTICLE SIZE SEPARATION AND SE SIZE SORTING USING ULTRASOUND IN A MICROCHANNEL" (Sergey Kapishnikov, Vasily Kantsler, Victor Steingberg), discloses two devices for separating and classifying particles and cells in suspension by means of ultrasound. The first of these is a so-called "lambda-half resonator" (lambda being the acoustic wavelength), wherein a standing wave with a pressure node in the centre of the channel is established, wheretowards the particles are transported and concentrated during application of the acoustic field and wherealong they continue circulating until reaching the exit. In this manner, the suspension enriched in ultrasonically concentrated particles abandon the device through a central outflow channel, while the rest of the suspension abandons the device mainly through another two lateral outflow channels symmetrically disposed with respect to the central channel. Inside the device, absolute separation of the acoustically collected particles from the rest of the elements present in the suspension, which circulate evenly distributed throughout the channel, is not possible. On not being affected by the ultrasonic field, their spatial layout is not altered during the application of the acoustic field, in such a manner that they abandon the device through the three outflow channels, even though they do so mainly through the lateral channels. This is because the ultrasonically concentrated cells exit the central channel en masse. In order to achieve selective separation, referred to as "cell classification" by the authors, the paper also presents the development of a lambda-quarter resonator wherein channel width is approximately a quarter of the wavelength and the pressure node is established next to one of the lateral channel walls, wheretowards the selected cells are transported and collected. As opposed to the present invention, this device requires the use of two ultrasonic transducers disposed on both sides of the treatment channel cross-section, external thereto, parallel and in opposition to each other, emitting temporary wave trains with a variable phase difference. This device, which most closely resembles that of the present invention, has significant differences which, as the case may be, give rise to certain operating restrictions, such as disabling the application of a continuous wave and physically limiting the action zone, which are determined by the space contained between the two sources of ultrasonic emission and the geometrically symmetrical layout of the inflow and outflow channels with respect to the central treatment channel, strongly limiting the ultrasonic action zone. As opposed to the present invention, this lambda-quarter device operates at around f = 4 MHz and is significantly restricted in terms of both treatment volume, the cross-section of which is inversely proportional to frequency (approximately four times less than those of our invention), and in the channel measurement accuracy required as a consequence of the foregoing.
■ Patent WO/2002/072236 PARTICLE SEPARATION (ERYSAVE AB, JÖNSSON Henrik, LAURELL Thomas, ALLERS Mats, PERSSON Hans), discloses a device for separating particulate matter (particularly for blood solution treatments) in a resonant device with a high number of acoustic pressure nodes for forming multiple particle concentration bands. It is a very different device to that of the present invention with regard to design and operation. The acoustic field is exerted on a treatment chamber that is very different to the lambda-half or lambda-quarter resonator channels, having much larger dimensions than these in order to enable establishment of a standing wave with spatial distribution of multiple nodes and maximum acoustic pressure values.
■ European patent EP1365849, also published as American patent US US6929750, US2004069717 and WO02072235, "DEVICE AND METHOD FOR SEPARATION", (LAURELL Thomas, ALLERS Mats, JÖNSSON Henrik, PERSSON Hans), discloses a microfluidic device for ultrasonic separation in half-wavelength resonators disposed in parallel forming a spatial distribution structure known as "array." Each of these elements contains a resonator channel called "lambda half" wherein the transport and acoustic collection of cells towards the centre thereof take place. As in other resonators of this kind, at the end of the process an enriched suspension of acoustically affected cells is achieved; however, it does not constitute an extraction process thereof with respect to the rest of the suspension, but rather a collection with a high concentration level through the central outflow channel. The device is embodied on an expensive silicon substrate customarily used in micro-devices of this kind. The patent envisages an increase in acoustic treatment volume by disposing multiple ultrasonic action channels in parallel. Each of these constituent elements of the device panel that forms what is technically known as an "array" contains a single entrance wherethrough the suspension is introduced and three outflow channels, a central channel wherethrough the suspension previously enriched in collected particles (erythrocytes) is discharged and two symmetrically disposed lateral channels, wherethrough the rest of the suspension circulates. Those elements not affected by the force of radiation remain homogeneously distributed in the sample and abandon the device through any of the three outflow channels, including (although to a lesser degree) the central channel wherethrough the erythrocytes are discharged en masse.
■ Patent WO 00/04978 CONCENTRATION OF PARTICLES IN A FLUID WITHIN AN ACOUSTIC STANDING WAVE FIELD, (CEFAI Joseph BARROW; David Anthony; COAKLEY William Terence; HAWKES Jeremy John, discloses a device for manipulating suspended particulate matter in a fluid. It contains a duct wherethrough the suspension circulates, in addition to an ultrasonic transducer and reflector for establishing a standing wave perpendicular to the direction of flow in the channel. The device is a "lambda-half resonator", with the formation of a single particle band in the centre of the duct. The distance between the transducer and the reflector is less than 300 microns, corresponding to a resonance frequency of the treatment chamber and not of the ultrasonic transducer. The device envisages a variation for this distance and for treatment frequency, in accordance with the construction tolerances of the device and the nature of the suspension to be treated. The suspension particles are collected and transported through the acoustic pressure node towards an exit disposed in a lateral channel of the device, wherethrough the particle-enriched suspension abandons the device. Meanwhile, the rest of the particles abandon the device through another outflow channel disposed with another orientation.
■ WO 98/50133 (WO 1998/050133) PARTICLE MANIPULATION, (COAKLEY William Terence; HAWKES Jeremy John, BARROW David Anthony CEFAI Joseph) discloses a device for manipulating suspended particulate matter consisting of a duct having a number of dimensions equivalent to a certain number of semi-wavelengths, wherethrough the suspension circulates and wherein a standing wave perpendicular to the direction of flow is established. A standing wave having multiple nodes and maximum acoustic pressure values is established in this treatment chamber for forming bands of cells which are acoustically collected, parallel, separated by a half-wavelength and perpendicular to the direction of circulation.
■ At IBM, Technical Disclosure Bulletin Vol. 25, No. 1, June 1982, page 192/193, discloses an ultrasonic continuous flow plasmapheresis separator. The device is composed of two orthogonally disposed transducers, each having a reflector and treatment volume therebetween in which to subject a diluted suspension to an acoustic standing wave. This treatment chamber is very different to that of the present invention, both in the form of transduction and in the treatment chamber.
■ JP 06241977 A discloses a device for measuring fine particles by means of an ultrasonic standing wave, acoustically induced by an ultrasonic beam in combination with an electrostatic force in order to concentrate and separate particles of different sizes. A pressure node is generated in the centre of the treatment chamber, wheretowards the particles are transported and concentrated.
■ EP0773055A2 (1996) (YASUDA K, SAITAMA H G, UMEMURA S, HUDRIOHI S) discloses a device and method for the acoustic manipulation of particles based on the interaction of several ultrasonic beams generated by different strategically disposed transducers. The suspension is subjected to an acoustic levitation process in a chamber, by means of an ultrasonic field generated by the interaction of several beams generated by multiple ultrasonic sources disposed in direct and/or indirect contact with the medium.
■ WO 93/19367A2 discloses an acoustic agglomeration method and device consisting of a tube containing a liquid sample and an ultrasonic transducer coupled thereto for generating a standing wave perpendicular to the tube, thereby concentrating the particles in multiple transversal bands, wherealong they sediment after the ultrasonic application. It is based on American patent numbers 5,665,605 and 5,912,182.
■ JP 07 047259A discloses a device for transporting small particles in suspension. The device contains multiple ultrasonic wave-generating elements disposed on a bidimensional transducer panel on two flat surfaces; wherebetween the solution to be treated is deposited.

### DESCRIPTION OF THE INVENTION

One aspect of the invention is constituted by a micro-device for selective and non-invasive separation and extraction of particles in polydispersed suspensions, hereinafter referred to as the micro-device of the invention, **characterized in that** it comprises the following components, integrated in a chip substrate of acoustically soft material:
a) a flow microchannel system having an asymmetrical spatial distribution of the outflow channelling branches stemming from the central treatment channel which comprises:
   i. a path or bed wherealong the starting suspension flows, which includes an inflow channel for supply and an outflow channel wherethrough it abandons the device, in parallel with
   ii. a path or bed wherealong the pure fluid wherefrom the selected particles will be extracted (called a fluid collector bed) flows, which includes an inflow or supply channel and an outflow channel that form
   iii. a central treatment channel where the starting suspension and pure fluid are separated by a border interface of streamlines under laminar flow regime defined by the transverse dimensions of the channel, which branches off into the two aforementioned outflow channels at the end of their path, where the width of the resonator channel - inversely proportional to acoustic frequency- is slightly larger at a quarter of the wavelength, and where the depth of the treatment channel (250 µm) -which forms its cross-section together with the width- is substantially smaller at a quarter of the acoustic wavelength,
   and
b) an ultrasonic actuator or transducer on one of the side walls
   - external and parallel to the central treatment channel, which transmits acoustic energy to the chip-channel assembly forming a multi-layer system, the resonance of which allows the pressure node to be correctly positioned inside the channel at a distance of approximately 1/3 and 2/3 of channel width, respectively, from the reflector wall, and the production of an acoustically generated pressure gradient inside the central channel which has the effect of transporting certain particles therewithin towards the acoustic equilibrium zone defined in the node, where the radiation force is annulled, and
   - the scope of action of which not only includes the central treatment channel, but also the region that branches off towards the two outflow microchannels, with the object of maximising selective separation and extraction efficiency.

One particular embodiment of the invention is constituted by the micro-device of the invention, wherein the constituent materials of the chip substrate, preferably an epoxy resin SU-8 whereon the channel is embodied and the acrylic PMMA (methyl polymethacrylate) substrate have an acoustic impedance of 3.3 MRayls, and where the ultrasonic transducer b) may be a small piezoelectric ceramic or rectangular piezoelectric composite, preferably a 1-3 class piezoelectric composite.

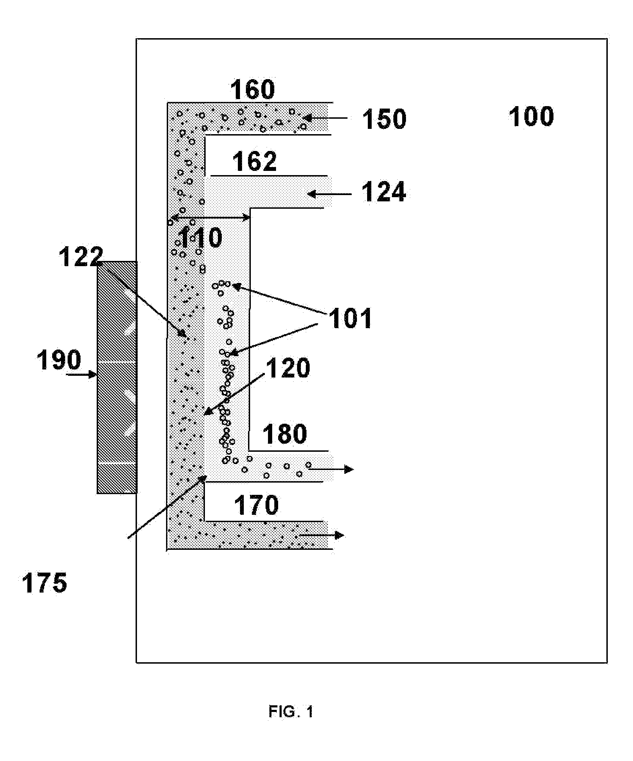

Another aspect of the invention is constituted by the manufacturing process of the micro-device of the invention, hereinafter referred to as the manufacturing process of the micro-device of the invention, based on the photolithography technique in accordance with the design described in Figure 1, which comprises the following stages:
a) Deposition and definition of a photodefinable polymer layer on the surface of an independent substrate (wafer 1),
b) Deposition and definition of a photodefinable polymer SU-8 layer on the surface of an independent substrate (wafer 2) covered by a non-stick material,
c) Sealing of wafer 1 and wafer 2, disposing said wafers in opposition to each other on the side containing the photodefinable polymer material, and
d) Removal of the wafer covered by non-stick material.

Therefore, another aspect of the invention is constituted by the use of the micro-device of the invention, hereinafter referred to as use of the invention, in a process for the selective and non-invasive separation, washing and/or classification of particles in polydispersed suspensions.

Another more particular aspect of the invention is constituted by the use of the invention wherein the particles consist of cells belonging, by way of example and without limiting the scope of the invention, to the following group: virus, prions, prokaryotic (bacteria, yeasts, fungi, algae, etc.) and eukaryotic cells.

A more particular embodiment of the invention is constituted by the use of the micro-device of the invention for the selective separation and isolation of eukaryotic cells, preferably human cells and, more preferably, tumour cells, blood cells (erythrocytes, platelets, macrophages and lymphocytes), stem cells or parent cells, whether somatic or embryonic or of another kind present in body fluids, such as for example: blood, urine, cerebrospinal liquid or present in other types of biological samples from biopsies.

A micro-device has been developed for the ultrasonic separation and extraction of particles and cells in suspension by means of a multi-layer-type ultrasonic resonator having a modified lambda-quarter channel with singular characteristics. Specifically, it has a particular geometric configuration, both in terms of the central treatment channel and the asymmetrical spatial distribution of the sample inflow and outflow channels with respect to the central treatment channel. The inventors have also discovered the importance of this last characteristic, which reinforces separation effectiveness, as described later in the text.

The present invention is based on the fact that the inventors have observed that the application of an activation wave generated by a transducer in parallel with a central treatment channel produces a standing wave therein perpendicular to the direction of flow, with a pressure node disposed in an intermediate position between the centre of the channel and the reflector wall in the region occupied by the pure fluid bed, which occupies the length of the acoustically affected treatment channel.

The device is activated by means of an ultrasonic actuator or transducer, for example a small, rectangular, piezoelectric ceramic or 1-3 class piezoelectric composite having a very low surface vibration amplitude, less than 10% at the ends and practically null in the middle. Said piezoelectric composite is formed from piezoelectric fibres embedded in a polymer matrix to constitute a composite of, for example, 1-3 class. In this manner, the coupling between the lateral modes associated with its dimensions is minimised, together with transmission thereof through the chip to the channel and therein.

One of the characteristics of the invention is that the ultrasound source is disposed in contact with the chip, on one of the outer edges, parallel to the treatment channel, to transmit the acoustic energy through its thickness in a direction perpendicular to the length of the treatment channel. The piezoelectric element is partially glued on one of its metallised surfaces to one of the outer edges of the chip of the micro-device, particularly the edge nearest the central treatment channel embodied on the chip, parallel thereto. In this manner, it transmits the acoustic energy through the successive layers that form the multi-layer system, establishing a standing wave with a pressure node inside the treatment channel, perpendicular to the direction of flow.

The location of the treatment channel with respect to the device assembly is important but not decisive; specifically, the inventors have developed two chip configurations with two distances from the channel to the edge of the chip where the ultrasonic actuator is disposed to improve pressure node stability therewithin:
- a first configuration with a distance of 750 µm (equivalent to three-eighths of a wavelength) between the treatment channel and the ultrasonic actuator;
- a second configuration with a distance of 500 µm (equivalent to a quarter of a wavelength) between the treatment channel and the ultrasonic actuator.

Both devices achieve an effective separation and extraction process, although certain optimisation of node stability in the channel can be observed in the second configuration. In the resonance of the multi-layer system, each and every one of the components (composite-chip-channel-chip) is involved in the establishment of the node inside the channel.

Therefore, the standing wave generated through the successive layers of the device is redistributed with respect to the first configuration, optimising the position of the pressure node inside the channel and enabling optimisation of the acoustic energy of the device. As opposed to our device, most technological developments are symmetrical and do not allow this possibility, given that resonance is formed inside the channel and the external layers are not so influential. The configuration versatility of our multi-layer system considerably increases the operating parameters for possible technological enhancements.

An acoustic pressure gradient is generated on the lateral walls of the channel, around the node disposed in an intermediate position between the centre of the treatment channel and the reflector walls, in the region occupied by the pure fluid bed throughout the length of the acoustically affected channel. This pressure node is produced at a distance of approximately 1/3 of the channel width from the reflector wall and 2/3 of the channel width from the opposite wall, respectively. Therefore, the pressure distribution established in this perpendicular direction to sample flow throughout the length of the channel originates a radiation force that acts in a specific manner on each suspended particle, perpendicularly to the direction of flow. However, its transport effect is limited only to those particles with a certain size, density or compressibility which, being susceptible to the acoustic conditions applied and selected in each case according to the specific type of application, are accelerated by the action of said force.

The strategic location of this acoustic equilibrium zone that constitutes the pressure node in the pure fluid bed, obliges the acoustically dragged particles to cross the separation interface between the two fluid media, thereby abandoning the suspension to be collected in the pure fluid, also hereinafter referred to as collector fluid, wherefrom they are extracted through one of the outflow channels.

The inventors also point out the importance of the strategic location of the node, relatively far from the reflector wall, as it represents an innovation with respect to "lambda-quarter resonators", wherein said node is located next to the reflector wall. This prevents problems caused by the adherence of the particles to said wall and favours the concentrated circulation thereof towards the channel exit, thereby avoiding obstruction problems. Additionally, the pressure node occupies a length along the channel similar to that of the ultrasonic actuator (length occupied by the piezoelectric ceramic or 1-3 class piezoelectric composite).

Another novelty of the present invention relates to the asymmetrical layout of the inflow and outflow channels stemming from the central treatment channel, which allows the ultrasonic transduction system to exert its influence through the chip on a wider area of the channel than that usually affected in these types of separators, which includes branching. This strategic spatial layout increases the possibility of disposing the ultrasound source (gluing area of the piezoelectric ceramic or piezoelectric composite) on the chip substrate, together with the action zone in the treatment channel, including the region that branches off towards the two outflow branches. In this region, geometrically different to the rest of the channel, the resulting radiation force is directed towards the channel exit wherethrough the particle collector fluid abandons the device, increasing selective separation efficiency.

This widening of the acoustic action zone ensures that the selected particles flow out through the desired channel, optimising separation and extraction effectiveness thereof from their initial medium, the suspension.

Another characteristic of this invention is the ultrasonic treatment frequency, 1 MHz, less than usual for micro-devices of this kind (which normally operate at a minimum of 2 MHz, most above this value). However, this frequency may vary, conveniently scaling the transversal dimensions of the central treatment channel, which must vary proportionally to the changes produced in wavelength (inversely to acoustic frequency). Therefore, low frequencies allow handling of greater treatment volumes. However, in the case of frequencies below 500 kHz, the acoustic cavitation threshold (which consists of the generation of micro-bubbles with strong and fast implosion effects in the medium) is also lower than that of higher frequencies. Due to this, the acoustic energy variability range for generating ultrasonic transport without causing damage to the suspended microelements is more restricted. Additionally, it must be taken into account that the radiation force increases linearly with acoustic frequency, due to which the volume benefits reported by the use of resonator devices at low frequencies have the drawback of higher energy consumption. In the case of the micro-device of this invention, ultrasound was applied at a lower than usual frequency, specifically at 1 MHz, for which, however, the acoustic cavitation threshold is high, demonstrating its viability and allowing a treatment volume at least twice as large as in the case of devices intended for resonation at 2 MHz. Therefore, the invention provides two advantages related to this acoustic parameter with respect to existing resonator micro-devices at higher frequencies; these refer to an increase in the aforementioned treatment volume and, consequently, a decrease in the restrictions associated with measurement adjustment precision (basically channel walls).

Another novelty of the micro-device of the invention is its constituent material, a chip integrated by two parallel-coupled materials: PMMA (methyl polymethacrylate) used as the constituent base substrate of the channel bottoms (with a thickness of approximately 900 µm) and a lamina of photodefinable epoxy SU-8, disposed on said substrate (with a thickness of 330 µm), whereon the channel is embodied. In this regard, special reflectors or similar have not been used for the walls of the central treatment channel and, on the contrary, the good behavior of the SU-8 material has been confirmed, which in the device constitutes a substantial part of the resonant multi-layer system. Its low acoustic impedance allows coupling of its resonant modes to those of the channel, without requiring special reflectors or similar for the treatment channel walls. Therefore, use of the polymeric material SU-8 as a reflector element for establishing the standing wave inside the channel and the advantageous applicability of these acoustically soft acrylic materials have been experimentally validated. Additionally, the good acoustic behavior of the constituent material of the channel bottoms, PMMA, has been verified as being a transmitter of ultrasonic energy with acoustic characteristics similar to those of SU-8 and good mechanical-acoustic coupling thereto. They are two polymeric materials which are easy to handle and low in cost. Both materials have low acoustic impedance (not higher than three times that of water and at least five times lower than that of metal) and allow easy handling thereof for creating the channels, in addition to the evident advantage of their lower cost compared to other substrates used in micro-devices of this kind, such as silicon, which is much more rigid from an acoustic viewpoint and more expensive. Overall, they offer interesting economic advantages.

The model used for experimentation, which is described in the second practical embodiment, is a model formed from polystyrene microparticles of different sizes and densities which could, for example, mimic the physical and acoustic characteristics of two types of cells: erythrocytes and tumor cells exfoliated from peripheral blood, initially flowing together in a fluid similar to blood plasma, in addition to any other sample containing microelements of these characteristics.

Worthy of mention is the high effectiveness of the selective separation and extraction of the particles with the greatest diameter obtained in the experiments carried out using the device of the invention. The repetitive behavior is due to the individual action of the acoustic radiation force on each particle, regardless of their concentration in the suspension. The effectiveness of the action is valid for both high concentrations and extremely diluted suspensions, where other separation techniques show a sharp reduction in action sensitivity and effectiveness.

In summary, of all the previously described novelties, the simplicity and effectiveness of the micro-device stand out: simplicity due to both the ultrasound source (consisting of a piezoelectric ceramic or piezoelectric component) and the geometry of the treatment channel and its inflow and outflow branches, in addition to the constituent materials of the chip of the device: plastic materials SU-8 (whereon the channel is embodied) on a PMMA substrate that constitutes the channel bases, in addition to its effective results.

Therefore, one aspect of the invention is constituted by a micro-device for the selective and non-invasive separation and extraction of particles in polydispersed suspensions, hereinafter referred to as micro-device of the invention, **characterized in that** it comprises the following components, integrated in a chip substrate of acoustically soft material:
a) a flow microchannel system with an asymmetrical spatial distribution of the outflow channelling branches stemming from the central treatment channel which comprises:
   i. a path or bed wherealong the starting suspension flows, which includes an inflow channel for supply and an outflow channel wherethrough it abandons the device, in parallel with
   ii. a path or bed wherealong the pure fluid wherefrom the selected particles will be extracted (called a collector fluid bed) flows, which includes an inflow or supply channel and an outflow channel that form
   iii. a central treatment channel where the starting suspension and pure fluid are separated by a border interface of streamlines under laminar flow regime defined by the transverse dimensions of the channel which branches off into the two aforementioned outflow channels at the end of their route, where the width of the resonator channel - inversely proportional to acoustic frequency- is slightly larger at a quarter of the wavelength, and where the depth of the treatment channel (250 µm) -which forms its cross-section together with the width- is substantially smaller than a quarter of the acoustic wavelength,
   and
b) an ultrasonic actuator or transducer on one of the side walls
   - external and parallel to the central treatment channel, which transmits acoustic energy to the chip-channel assembly forming a multi-layer system, the resonance of which allows the pressure node to be correctly positioned inside the channel at a distance of approximately 1/3 and 2/3 of channel width, respectively, from the reflector wall, and production of an acoustically generated pressure gradient inside the central channel which has the effect of transporting certain particles therewithin towards the acoustic equilibrium zone defined in the node, where the radiation force is annulled, the scope of action of which not only includes the central treatment channel, but also the region that branches off towards the two outflow microchannels, with the object of increasing selective separation and extraction efficiency to a maximum.

Use of the term "particle" in "polydispersed suspensions" in the present invention refers to a suspension with particles of different physical characteristics (size, density or compressibility, among others), comprising inorganic or organic microelements such as cells, preferably eukaryotic cells, more preferably human cells, microorganisms or other types of microelements present in biological fluids with parameters of the same order.

Use of the term "chip made of acoustically soft materials" refers to materials with an impedance far below that of other materials or media such as metals or glass (at least five times lower) and, fundamentally, no more than three times the impedance of liquid media (usually delimited within a variability range that generally varies, save for exceptions, between 0.8 MRayls and 2.6 MRayls). The concept of "soft" therefore refers to the impedance relationship between the constituent material of the treatment channel walls and the fluids circulating therewithin, but having sufficient capacity to produce reflections of the acoustic wave to establish standing waves.

Therefore, any soft material, preferably an acrylic material, having acoustic properties similar to SU-8 or other plastic elements may be used as a material for manufacturing the chip substrate of the micro-device of the invention whereon to embody the channel, due to its similarity in terms of transmission of acoustic energy therethrough and similar reflection responses on the channel walls.

A particular embodiment of the invention is constituted by the micro-device of the invention, wherein the constituent materials of the chip substrate, preferably epoxy resin SU-8 whereon the channel is embodied and the acrylic substrate PMMA (methyl polymethacrylate), have an acoustic impedance of 3.3 MRayls, and where the ultrasonic transducer b) may be a small piezoelectric ceramic or piezoelectric composite, preferably one of 1-3 class.

Another aspect of the invention is constituted by the manufacturing process of the micro-device of the invention, hereinafter referred to as manufacturing process of the micro-device of the invention, which is based on the photolithography technique in accordance with the design described in Figure 1, which comprises the following stages:
a) Deposition and definition of a photodefinable polymer layer on the surface of an independent substrate (wafer 1),
b) Deposition and definition of a photodefinable polymer SU-8 layer on the surface of an independent substrate (wafer 2) covered by a non-stick material,
c) Sealing of wafer 1 and wafer 2, disposing said wafers in opposition to each other on the side containing the photodefinable polymer material, and
d) Removal of the wafer covered by non-stick material.

The present micro-device can be easily manufactured by a person skilled in the art with the knowledge and designs indicated in the present invention and with the current state of the art. Additionally, the design of the micro-device of the invention can be enhanced by introducing additional empty channels strategically disposed around the central channel to minimize the loss of acoustic energy transmitted through the PMMA chip substrate and SU-8 material. These additional elements may easily be incorporated in the design of the device of the invention by repeating steps b), c) and d) of the manufacturing process of the device and adding two sealed air-filled channels beneath the central channel and parallel thereto.

There is an air-filled channel disposed both beneath and next to the fluidic treatment channel where the separation takes place. In this manner, the ultrasound signal used for separation is disposed in the desired confined position, thereby minimizing losses. The configuration of the central channel can also be enhanced by:
- varying its position with respect to the micro-device assembly in order to enhance the stability of the pressure node inside the channel. Specifically, by varying the distance between the channel and the outer wall of the chip next to the ultrasonic actuator, the distance can be reduced up to a quarter of a wavelength in said medium (for which the transmission of acoustic energy is maximum at the selected frequency). Therefore, the stability of the pressure node inside the channel (wheretowards the microelements are transported and acoustically collected) is favored.
- reducing the length of the treatment channel by half, in addition to that of the whole device in the same proportion. Shortening the distance travelled by the two samples flowing parallel to each other inside the treatment channel reduces their interface in the same proportion. Consequently, diffusion path length is shortened considerably. In fact, in non-Newtonian fluids such as for example blood (the viscosity of which varies depending on certain parameters), channel shortening is essential to avoid interface rupture and prevent them from invading the whole channel. On the other hand, channel shortening implies less residence time of the fluid therein and, therefore, less acoustic treatment, in addition to greater manageability.

Additionally, the operation of the micro-device can be enhanced by slightly modifying operating frequency, as the system shows well-differentiated micro-manipulation capabilities making slight variations in frequency around the core operating frequency for which it was designed. Increases in frequency of less than 12% of its core value allow modification of the equilibrium position and collection of the microelements inside the channel towards the desired position in accordance with the application to be developed. This characteristic gives the micro-device broad application versatility.

On the other hand, the operation of the micro-device can be enhanced by broadening the operating frequency range, as the system has micro-manipulation capabilities by making slight variations in frequency around the core operating frequency for which it was designed. Increases in frequency of less than 12% of its core value allow modification of the equilibrium position and collection of the microelements inside the channel towards the desired position in accordance with the application to be developed. This characteristic gives the micro-device broad application versatility.

On the other hand, the micro-device of the invention can also be manufactured using hot-stamping techniques combined with a subsequent gluing process, in the following manner:
a) Preparation of a mould wherein the desired channel designs are included,
b) Molding of the substrate to be used, using the mould obtained in a) under the action of pressure and/or temperature, and
c) Sealing of the substrate by gluing to another plastic material under the action of pressure and/or temperature and/or oxygen plasma surface activation.

On the other hand, the frequency range applicable to the micro-device of the invention for both organic and inorganic suspensions is broad, although certain considerations must be taken into account in the case of organic suspensions, as explained hereunder. One variation in ultrasonic frequency implies a scaling process in the dimensions of the device. Given that operation of the micro-device is based on the acoustic resonator model in the direction of channel width, the spatial characteristics associated with this lateral dimension of the treatment micro-channel must be varied in inverse proportion to the acoustic frequency. Although the radiation force induced on each micro-element of the suspension is directly proportional to the frequency, the decrease in the acoustic cavitation energy threshold must be taken into account in the case of organic suspensions with low frequency levels (in the order of kHz) so as to avoid cell damage. This undesired phenomenon is favored by low frequencies, due to which there would be limitations to the application of the invention below 500 kHz. On the contrary, the increase in frequency linearly increases the magnitude of the radiation force and allows a reduction in the acoustic energy levels required to generate selective ultrasound-based transport. For this reason, nearly all the devices developed to date operate at between 2 MHz and 5 MHz. In contrast, an increase in these frequencies implies a scaled reduction in the lateral dimensions of the treatment channel, which must vary proportionally to the changes induced in the acoustic wavelength, raising the cost of the manufacturing processes of these devices due to the need for precision.

The results obtained using this model allow application of the device in the sphere of particle separation and isolation, with important applications in agrobiotechnology, biotechnology applied to human and animal health such as, for example, separation and isolation of cells, preferably human, and diagnostic and treatment processes, for example, cell or gene therapy treatment of mammal diseases, preferably those of human beings.

Therefore, another aspect of the invention is constituted by the use of the micro-device of the invention, hereinafter referred to as use of the invention, in a process for the selective and non-invasive separation, washing and/or classification of particles in polydispersed suspensions.

Another more particular aspect of the invention is constituted by the use of the invention wherein the particles consist of cells belonging, by way of example and without limiting the scope of the invention, to the following group: virus, prions and both prokaryotic (bacteria, among others) and eukaryotic cells.

A more particular embodiment of the invention is constituted by the use of the micro-device of the invention for the selective separation and isolation of eukaryotic cells (such as algae, fungi -including yeasts-), preferably human cells and, more preferably, tumour cells, blood cells, stem cells or parent cells, whether somatic or embryonic or of other kinds present in body fluids, such as for example: blood, urine, cerebrospinal liquid or those present in other types of biological samples from biopsies.

Specific biomedical processes or applications, whether in relation to diagnosis or treatment, where the micro-device of the invention can be used are those related to blood donations, plasmapheresis, dialysis processes and laboratory analyses, in addition to recycling and/or washing of blood after surgical operations, where the separation and concentration of certain types of cells, for example erythrocytes and platelets, is required.

Another example is constituted by the use of the micro-device of the invention in a human disease diagnosis and/or treatment process for the selective separation and extraction of damaged or altered cells of patients, which can be repaired ex vivo and re-administered to the patient.

A specific field of biomedical application is oncology, where it can be used as a diagnostic and prognostic tool for reproducing the selective separation and extraction of circulating tumour cells in peripheral blood (CTC) of oncology patients with solid tumours of different tissular origin and at different stages of the disease.

The clinical use demonstrated to date in the quantification of the number of circulating tumour cells in peripheral blood focuses on the following aspects:
- Independent prognostic factor in breast and metastatic prostate cancer *(*Cristofanilli M, Budd GT, Ellis MJ, Stopeck A, Matera J, Millar MC, Reuben JM, Doyle GV, Allard WJ, Terstappen LW, Hayes DF. Circulating tumour cells, disease progression and survival in metastatic breast cancer N Engl J Med 351:8, 2004*) (*Moreno JG, Milelr MC, Gross S, Allard WJ, Gomella LG, Terstappen LW. Circulating tumour cells predict survival in patients with metastatic prostate cancer. Urology; 65(4):713-718; 2005*).*
- Monitoring of the response shown by oncology patients with an advanced stage of the disease to chemotherapy treatments *(*Cristofanilli M, Mendelsohn J, et al. Circulating tumour cells in breast cancer: advanced tools for tailored therapy. Proc Natl Acad Sci USA 103(46):17073-17074; 2006*).*

The analysis systems used in these studies are based on positive immunomagnetic separation using monoclonal antibodies and subsequent analysis using fluorescence microscopy. These applications have obtained the approval of the Food and Drug Administration (FDA) for use thereof in clinical practice in the United States.

The use of both applications for other types of tumours is becoming widespread. Likewise, there are preliminary studies that indicate the potential use of the analysis of the number of CTC as an early marker for relapses in colorectal cancer *(*Soto JL, Garrigos N, Gallego J, Guaraz P, Garcia-Bautista M, Castillejo A, Gomez A, Casado-Llavona C, Rodríguez-Lescure A, Carrato A. Toward a circulating tumour cell analysis as an early marker for relapse in stage II and III colorectal cancer patients. Eur J Cancer Supplements. 3(2):187; 2005*).*

One of the main advantages of the device of the present invention is the real possibility not only of effectively separating CTC -which would allow easy counting thereof- but also of being able to isolate said cell population in viable conditions for subsequent analyses -both descriptive on a genetic level and gene expression profiles- and ex vivo functional behavior studies. To date, it is the only known device capable of offering said possibility with such high effectiveness.

The concept of CTC as an affordable and non-invasive tumour biopsy has the added value of the possibility of functionally characterizing the behavior of said cells with respect to their sensitivity/resistance to the available therapeutic arsenal as a personalized system for selecting the most effective treatments for each patient.

The real and potential clinical use of the device is therefore of great importance to clinical practice, offering highly valuable information for better managing patients with different diseases.

### DESCRIPTION OF THE FIGURES

Figure 1 shows a perspective (2D) schematic view of the elements and manner in which the micro-device of the invention acts upon/transports suspended particulate manner.
Figure 2 shows a multi-layer configuration of the device of the invention.
Figure 3 shows a photograph of the prototype of the device with the chip and piezoelectric ceramic transducer integrated in an assembly piece for the insertion/extraction of fluids.
Figure 4 shows a photograph of the prototype of the device with the chip and piezoelectric composite transducer, integrated in an assembly piece for the insertion/extraction of fluids.
Figure 5 shows a photograph and diagram of the PMMA chip.
Figure 6 shows microscopic photographs of the interior of the channel.
Figure 7 shows a microscopic photograph of the individual displacement behaviour of each 20 µm particle towards the acoustic pressure node inside the channel.
Figure 8 shows a photograph of 20 µm particles.
Figure 9 shows a filming of particle separation/extraction.
Figure 10 shows the extraction process of 20 µm particles through the outflow channel.
Figure 11 shows a diagram of the manufacturing process of the micro-device of the invention using a photodefinable material as structural material.
Figure 12 shows the design of the micro-device of the invention where channels sealed at their ends and filled with air are strategically disposed with respect to the central channel in order to minimise energy loss during the transmission process through the substrate. **a)** Chip seen from above, **b)** Cross-section of the chip.
Figure 13 shows a diagram of the manufacturing process of the micro-device of the invention using the hot-stamping technique.
Figure 14 shows the action and control capacity of the micro-device in its acoustic action on the microelements inside the channel, by means of slight variations in the frequency of its core value, which allows modification of the equilibrium position and collection of microelements inside the channel (930 kHz) up to the reflector walls (1.1 MHz) towards the desired position in accordance with the application to be developed.

### PRACTICAL EMBODIMENTS OF THE INVENTION

The first practical embodiment describes a first prototype of the micro-device of the invention.

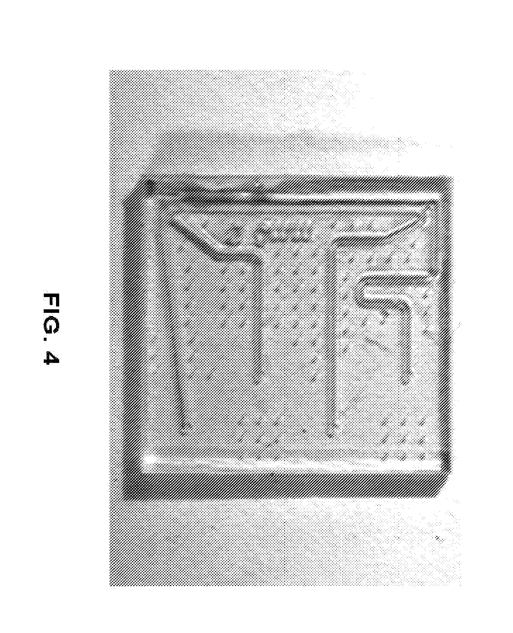

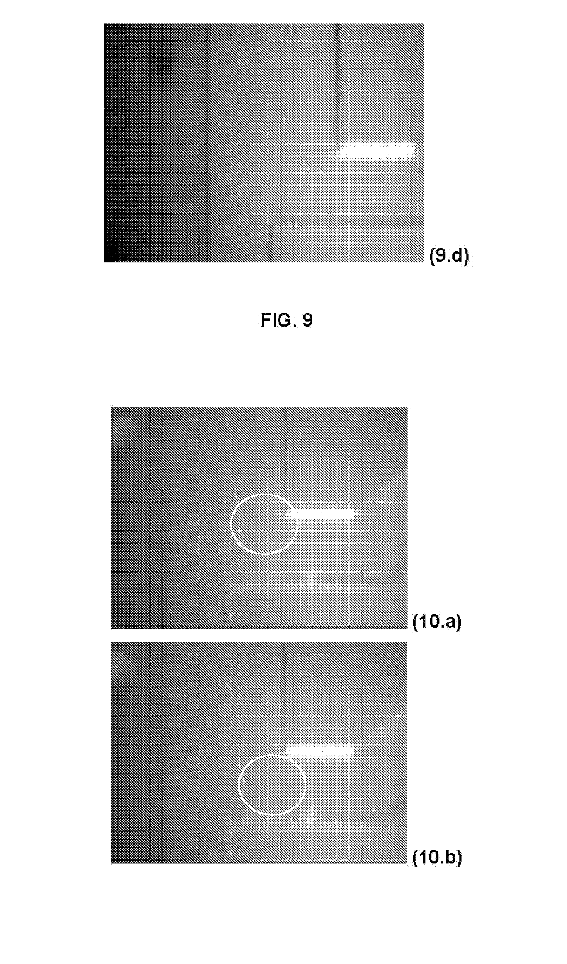

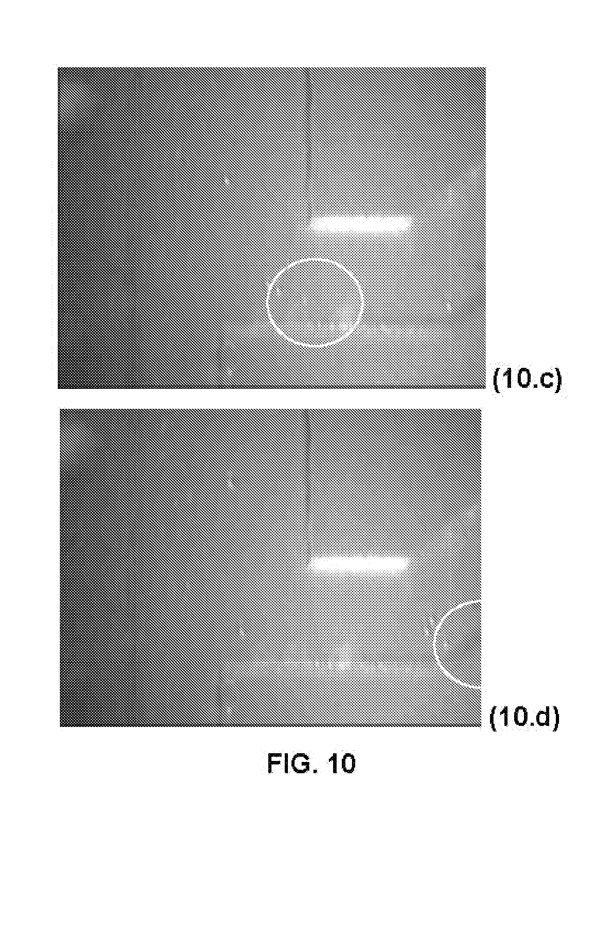

In the prototype shown in Figure 4, the structural material used to embody the fluidic channels was photodefinable polymer SU-8, mechanically coupled to a PMMA substrate, which constitutes the bottom of the channels of the device. This material has very convenient properties for manufacturing devices due to its high definition (at micrometre scale), verticality in the developed walls [ref2], biocompatibility [ref3], broad range of thicknesses [ref1] and possibility of gluing several consecutive layers [ref4]. The manufacturing procedure of this prototype of the device of the invention used, in accordance with the design of Figure 1, is the following (see Figure 10):
a) Deposition and definition of a photodefinable polymer layer on the surface of an independent substrate (wafer 1),
b) Deposition and definition of a photodefinable polymer layer SU-8 on the surface of an independent substrate (wafer 2) covered by a non-stick material,
c) Sealing of wafer 1 and wafer 2, disposing said wafers in opposition to each other on the side containing the photodefinable polymer material, and
d) Removal of the wafer covered by non-stick material.

The prototype of the micro-device has been designed and manufactured in such a manner as to comprise a chip (100) with an integrated system of four micro-channels (160, 162, 170 and 180), centred around a central treatment channel (110), two on either end thereof, asymmetrically disposed, for both inflow and outflow of two media circulating in parallel under laminar regime along the channel (110) (see figures 1, 2 and 3). One of these media is the suspension (150) wherefrom certain particles will be extracted (101) and the other is a pure liquid (124) that will act as an ultrasonic collector of the particles (101).

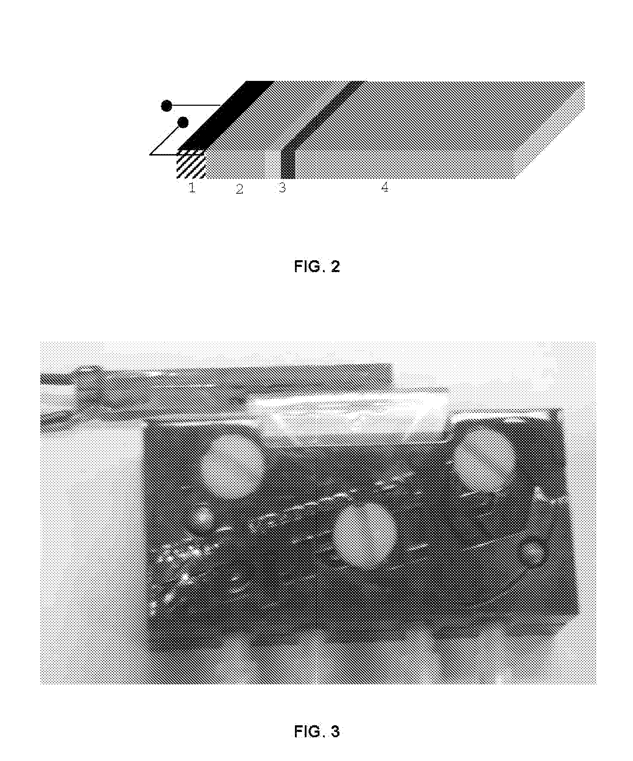

As can be observed in Figure 2, an ultrasonic transducer (190) glued to one of the metallised surfaces on the edge of the chip includes two polymeric materials (one with channelling and another that constitutes the substrate whereto it is mechanically coupled) and generates an ultrasound wave which it transmits through the acrylic chip to the treatment channel. Channel width "w" is approximately a quarter of the acoustic wavelength and allows establishment of a standing wave in said direction inside the channel with a pressure node at a distance of approximately w/3 with respect to one of its lateral walls, which acts as a reflector.

Photograph 3.a shows the device from above and photograph 3.b shows the chip structure edgewise with the two mechanically coupled polymeric materials.

As can be observed in the photograph of Figure 4, the device has an ultrasonic actuator or transducer integrated in one of its sides (190), which can consist of a small, rectangular PZ26 piezoelectric ceramic with thickness mode resonance at 1 MHz or a 1-3 class piezoelectric composite, partially glued by one of its metallised surfaces to the thickness of the chip made of SU-8 and the PMMA substrate by one of its lateral edges, partially occupying its thickness and disposed parallel to the treatment channel. The ultrasound source is disposed in perpendicular contact with the chip. Transmitting ultrasonic energy in a perpendicular direction thereto, in such a manner that transmission of acoustic energy to the medium inside the channel occurs perpendicularly to the direction of flow.

More specifically, channel width is 390 ± 4.6 µm (1.06 times a quarter of the wavelength for 1 MHz). The pressure node is disposed at a distance of 117 ± 4.6 µm from the reflector wall, in the region occupied by the pure fluid bed, external to the suspension. Therefore, the channel has a cross-section of 0.0975 mm² and a wavelength that can vary freely, although in the specific case of the invention it is 1 cm. Therefore, channel volume is 0.975 mm³.

Figure 10 shows the extraction of the particles through the outflow channel on the right after the individual ultrasonic transport thereof at an extremely low circulation speed of approximately 0.06 mm/s, which allows clear visualization and quantification of the acoustic behavior thereof. The consecutive photograms (10.a) to (10.d) clearly show the effectiveness of the ultrasonic selective separation treatment, wherein all the particles with a diameter of 20 µm abandon the device towards the pure collector fluid outflow channel, while the rest of the suspension, which contains the small 6 µm particles, circulating along its left "bed", is discharged through the corresponding channel.

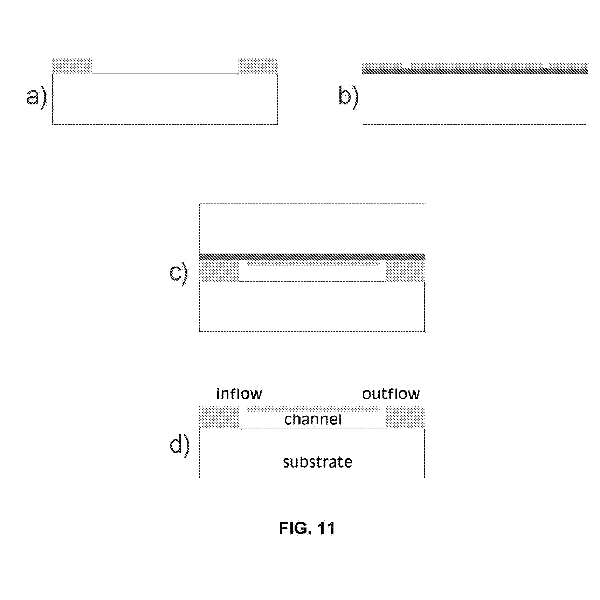

Figure 11 shows a diagram of the manufacturing process of the micro-device of the invention using a photodefinable material as structural material.
a) Deposition and definition of a photodefinable polymer layer on the surface of an independent substrate; b) Deposition and definition of a photodefinable polymer layer on the surface of an independent substrate covered by a non-stick material; c) Sealing of wafer 1 and wafer 2; and d) Removal of the wafer covered by non-stick material.

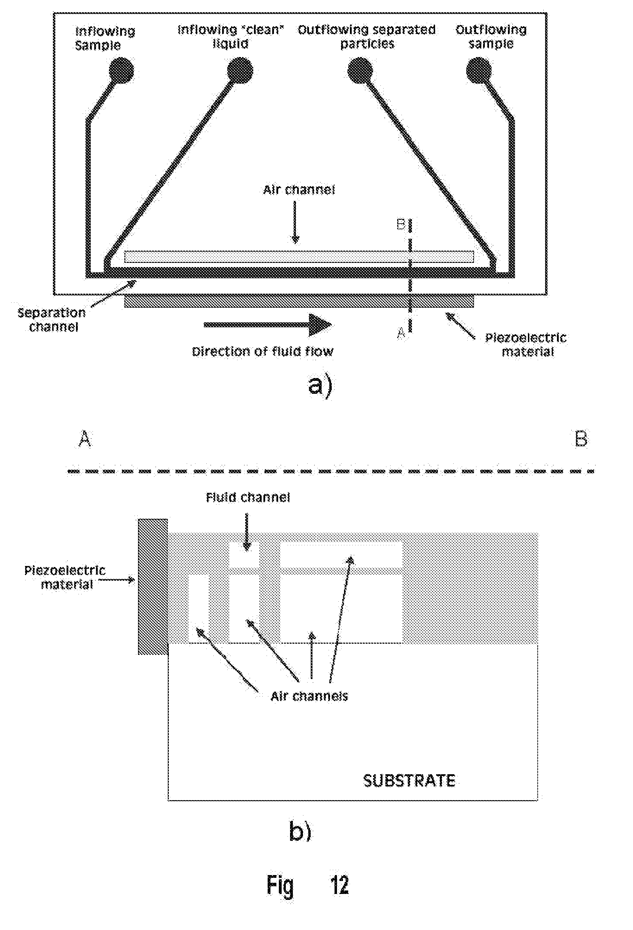

Figure 12 shows the design of the micro-device of the invention, wherein air-filled channels with sealed ends are strategically disposed with respect to the channel to minimize energy loss during the transmission process through the substrate. a) Chip seen from above, b) Cross-section of the chip.

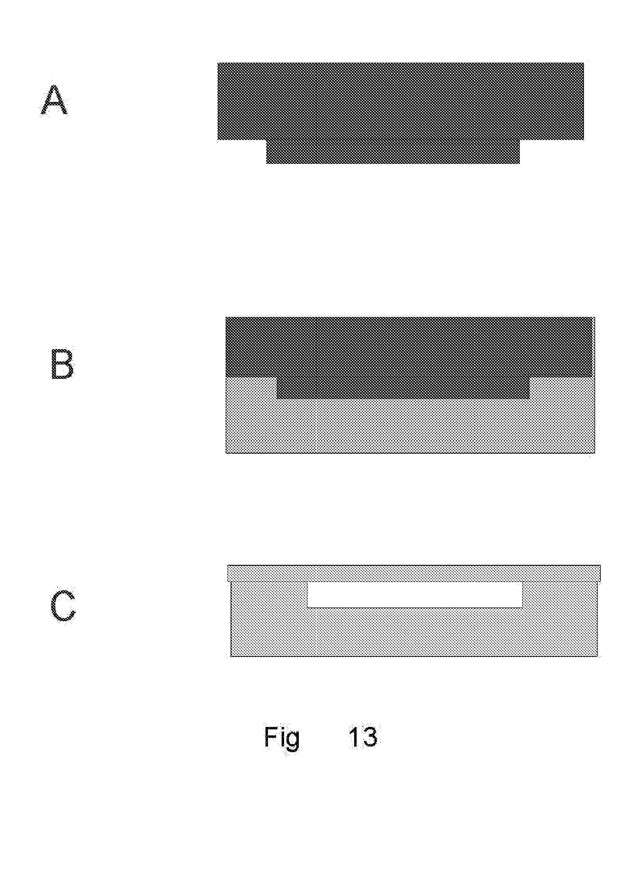

Figure 13 shows a diagram of the manufacturing process of the micro-device of the invention using the hot-stamping technique.
a) Preparation of a mould wherein the designs of the desired channels are included, b) Molding of the substrate to be used, using the mould obtained in a), under the action of pressure and/or temperature, and c) Sealing of the substrate by gluing to another plastic material under the action of pressure and/or temperature and/or surface activation by oxygen plasma.

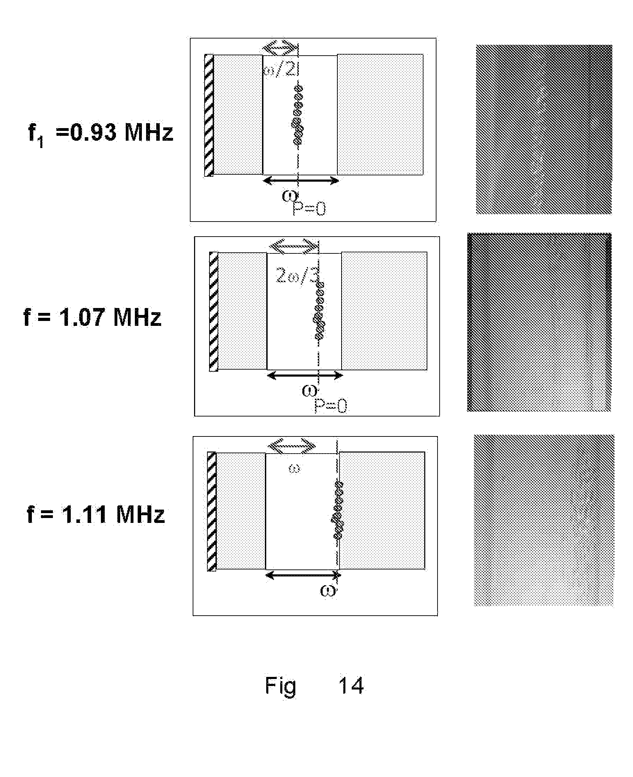

Figure 14 shows photographs that illustrate the action and control capacity of the micro-device in its acoustic action on the microelements inside the channel, by means of slight variations in its frequency value which allow modification of the equilibrium position and collection of microelements inside the channel (930 kHz) up to the reflector wall (1.1 MHz) towards the desired position in accordance with the application to be developed.

In the second embodiment, the use of the micro-device of the invention in the separation of cell-mimicking microparticles is described.

For the microfluidic control of the micro-device of the invention, a constant-pressure injection pump with simultaneous application capacity to three syringes of different volumes (between 10 µl and 110 ml) was used to control the flow of both media at each of the entrances (160 and 162). The suspension (150) and collector fluid (124) were simultaneously injected at the same pressure using syringes of the same volume (5 ml each) through these entrances (160 and 162), respectively.

The ultrasonic separation of the selected particles and transport thereof to the pressure node, in the collector fluid (124) bed, were monitored in real time using filmings of microscopic resolution made with a CCD camera coupled to an optical lens assembly with a resolution of 1.17 µm/digital pixel. The width of the fluidized bed occupied by the suspension was maintained at around 1/2 of the channel (110) width.

The model used for experimentation in this example is a polystyrene microparticle model with sizes and densities that mimic the physical and acoustic characteristics of two types of cells: erythrocytes and tumour cells exfoliated from peripheral blood, initially flowing together in a fluid similar to blood plasma.

The physical and acoustic characteristics of this fluid, such as density and acoustic propagation speed thereof, are described in the paper (Cousins CM, Holownia P, Hawkes JJ, Limaye MS, Price CP, Keay P, Coakley WT, Plasma preparation from whole blood using ultrasound. Ultrasound Med Biol 26:881-888, 2000), in addition to those of the erythrocytes (Duck F A, Physical properties of tissue: a comprehensive referente book, Academia London, 1990,Haider L, Snabre P, Boynard M, Rheology and ultrasound scattering from aggregated red cell suspensions in shear flow, Biophysical Journal, Vol. 87, 2322-2334, 2004). On the contrary, the non-existence of bibliographic references to these properties for circulating tumour cells in peripheral blood obliged us to determine said data indirectly through the development of an experimental induction model. To this end, we resorted to the acoustic characterisation of two liquid media frequently used in oncology laboratories for cell separation by centrifugation and density gradient: Ficoll® and another liquid medium not technically defined but used in an experimental device called Oncoquick®, which is very effective in the separation of tumour cells due to its density, whereon the tumour cells exfoliated from peripheral blood float [Rosenberg R, Gertler R, Friedrichs J, Fuehrer K, Dahm M, Phelps R, Trovan S, Nekarda H, Siewert JR. Comparision of two density gradient centrifugation systems for the enrichment of disseminated tumour cells in blood. Cytometry 49; 150-158. 2002].

The possible variability margin for tumour cell density was derived from the density and sound propagation speed measurements in both liquids: 1.030 gr/cm³ < p (tumour cells) < 1.055 gr/cm³, with an uncertainty degree of less than 5% of the minimum value. Based on these data and taking into account the approximately linear dependence for biological microelements, their compressibility was estimated selecting, for this example, particles with a density of 1.05 gr/cm³, as being representative of tumour cells.

Once the tumour cells were characterized using this experimental model, they were mimicked by polystyrene particles with selected diameters of 20 µm. Although the variability range of these cells is very broad (definable between 10 and 40 µm), this size was chosen as a standard value.

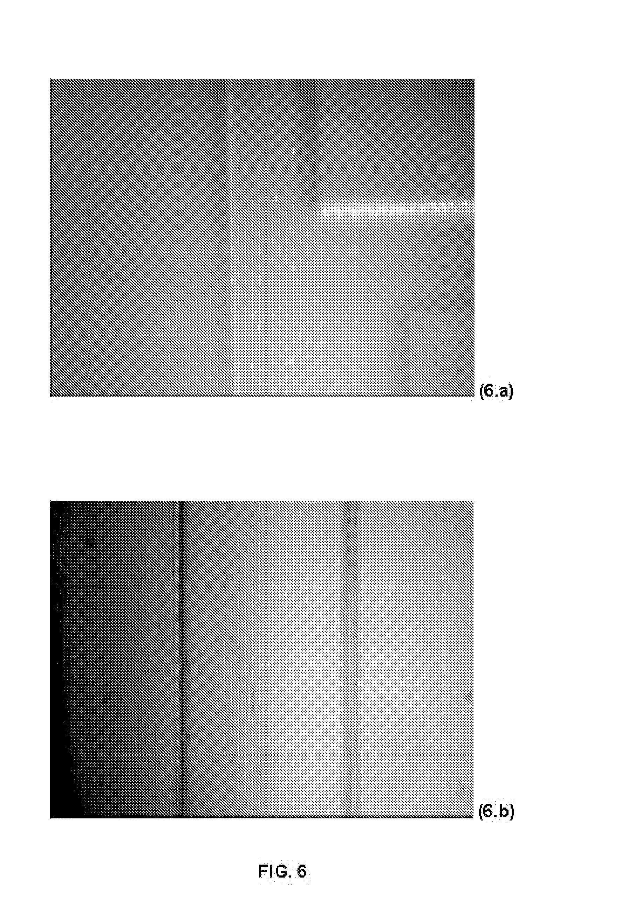

In this manner, we proceeded to introduce and analyses "bi-disperse" aqueous suspensions, i.e. containing two particle populations with a diameter of 6 and 20 microns, respectively, in different concentrations and subjected to ultrasound in the device. The results of the selective separation and extraction of the large particles can be clearly observed in figures 6, 7, 8 and 9.

In accordance with Figure 6, the occupation of the two media inside the channel can be observed: the suspension circulating along the left section of the channel and water in the right section, in the absence of the ultrasonic application. The suspension consists mainly of small polystyrene particles with diameters of 6 microns with a high concentration and some larger particles with diameters of 20 microns circulating at a very low concentration (Cv < 1 %). In the photograph of Figure 6.a) we can observe the transversal distribution of the media at low circulation speeds, allowing partial visualization of 6 µm. Image resolution is 1.17 µm/pixel. On the contrary, the photograph of Figure 6.b), corresponding to a higher circulation speed, does not allow said distinction but shows a different tonality in the luminous contrast between the two media.

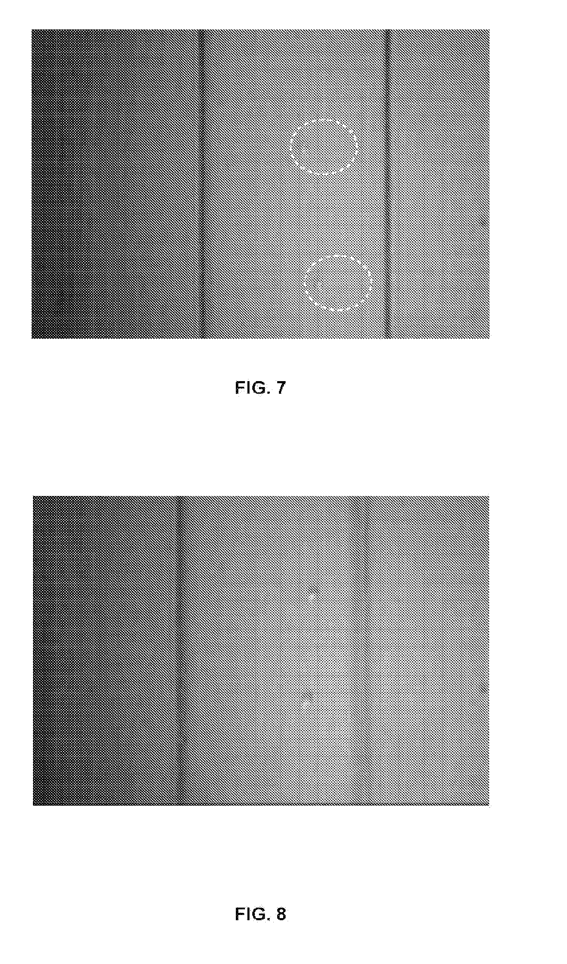

In accordance with Figure 7, the particle located on the upper part of the channel is at the start of the channel section affected by the ultrasonic actuator and undergoes lateral displacement, perpendicular to the direction of flow, less intense than the particle located at the bottom of the photograph, which is fully affected by the acoustic field and, as a result, dragged more intensely. For this reason, said particle is located in the position of the pressure node while the upper particle has still not reached it during the time of acquisition of the photogram.

Figure 8 shows an image corresponding to a photogram of two 20 µm particles circulating very slowly along the channel in the pressure node, positioned in the acoustic pressure node, separated from the reflector wall.

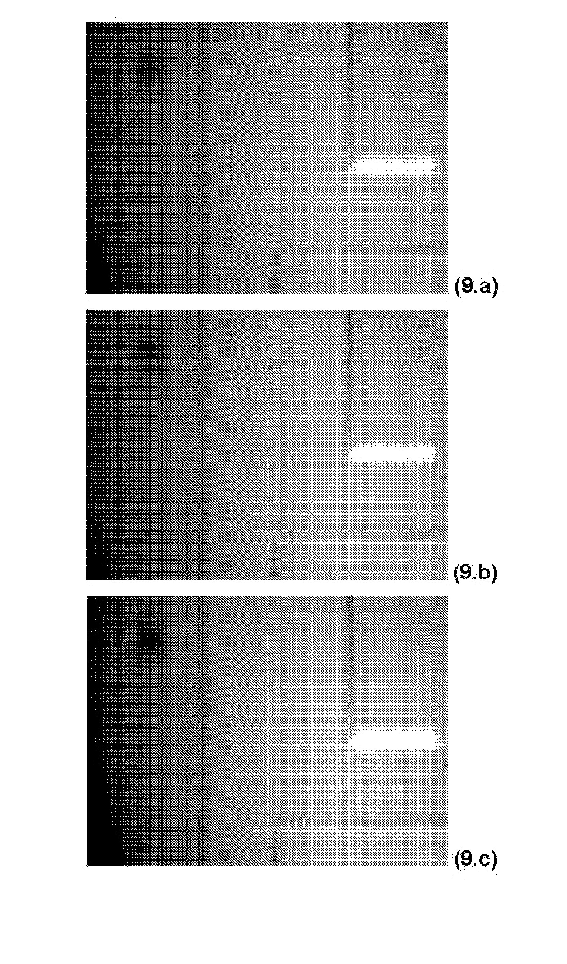

Figure 9 shows experimental results in a filming of the separation/extraction process of the selected particles through the outflow channels. The first photogram (9.a) shows, in the absence of ultrasound, the natural outflow of the 20 µm particles together with the rest of the suspension through the left branch from the channel, following the fluidized bed wherealong it circulated. The photograms of (9.b), (9.c) and (9.d) show the selective outflow of these particles, separated from the suspension, through the pure fluid-water outflow channel, once transported and acoustically collected in the pressure node, located on the pure fluid bed (right semi-section), wherealong they continue to circulate until abandoning the treatment channel. All of these photograms correspond to the same film. Particle circulation speed in these sequences is 2.4 mm/s throughout the channel.

The results of the selective separation of the 20 µm were positive for all the tests conducted on the samples injected through the central treatment channel (within a range of variability between 0.06 mm/s and 1.4 mm/s) at different flow speeds, always under the laminar regime required in microfluidics. Bi-disperse aqueous suspensions were used: with different volumetric concentrations of small particles (6 µm), not quantified, and large particles with diameters of 20 µm at a very low concentration, always less than 1%. All the experiments were conducted at the frequency determined by channel width, strategically determined based on the resonant frequency of the PZ26 piezoelectric ceramic: 1 MHz. Deionized water was always used as the pure fluid.

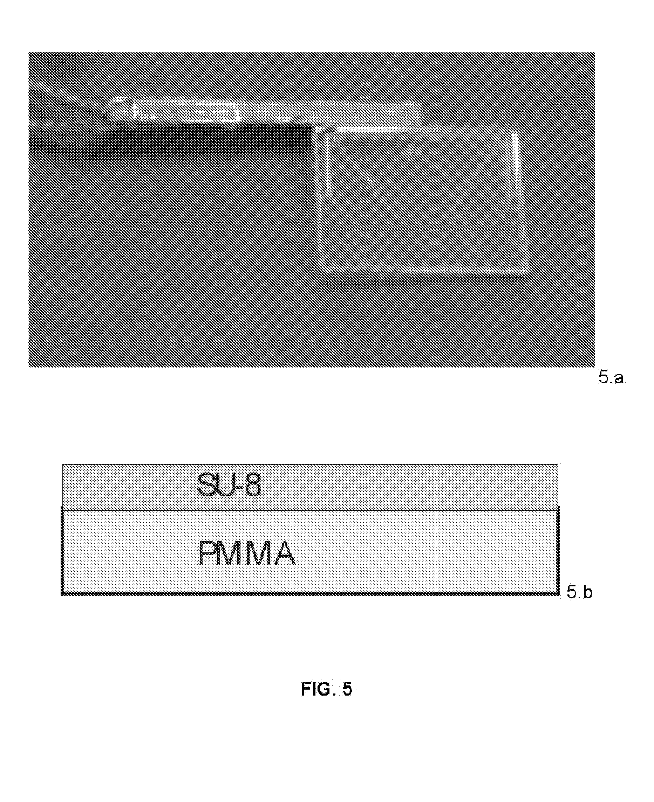

More specifically, the two media were introduced into the central channel in parallel: a suspension (150) wherefrom particles having certain characteristics (101) were extracted (specifically, particles with a diameter of 20 µm and a density of 1.05 gr/cm³) and a liquid fluid (deionized water) (124), through two channels (160 and 162), both having the same cross-section (0.049 mm²) and integrated in the chip of the invention, each of which occupy half of the section of the central channel (110). The two media flow through the channel (110) in parallel and under laminar regime along their corresponding bed: the pure fluid that will collect the particles (101) along a fluidized bed (124) and the suspension along the bed (122) that occupies the other part of the channel (110) section, keeping the interface that separates them (120) stable. This behavior can be observed in the photographs of Figure 5 at two continuous flow speeds (0.06 mm/s and 1.4 mm/s, respectively). Photograph **5.a** shows the embodied micro-channels and the position of the piezoelectric ceramic glued onto the edge of the chip. Figure **5****.b** shows a diagram of the cross-section of the chip, constituted by two polymeric materials SU-8 and PMMA. After covering the length of the channel (110), the two media are separated at the branching point (175) towards two outflow channels (170 and 180), wherethrough they abandon the device. When the suspension (150) flows along its bed (122) inside the channel (110), those particles of a certain size and density (101) contained therein are subjected to a radiation force due to the establishment of a standing wave generated in the channel (110) by the externally positioned piezoelectric transducer (190).

For supply voltages of 15 volts applied to the ultrasonic transducer from a continuous signal generator, the 20 µm particles (101) are subjected to a radiation force and are rapidly transported perpendicularly to the continuous flow of the suspension along the channel (110) under the action of the ultrasounds towards the pressure node, located in the region occupied by the pure fluid (water) (124) (Figure 6), wherethrough they continue circulating towards the end of the channel (Figure 7), abandoning the device in a differentiated manner through the outflow channel (180) immersed in said fluid (124) and separated from the rest of the suspension that contained them prior to the ultrasonic application.

On the contrary, the small 6 µm particles contained in the suspension (107) at a high concentration are not affected by the acoustic field and do not undergo acoustic dragging, given that the radiation force exerted thereupon is much smaller due to being proportional to the third power of the radius, which is three times smaller than that of the large particles (101). In this manner, the particles continue circulating in the suspension along their initial fluidized bed without altering their paths. Finally, they abandon the device through the suspension outflow channel.

Figures 8 and 9 show two collections of consecutive photograms wherein we can observe the circulation of the 20 µm particles once ultrasonically extracted from their suspension and collected in the collector fluid towards the branching point of the central channel, wherefrom they abandon the device through the channel (180), separated from their initial medium. Given the width of the channel (110), just over a quarter of the acoustic wavelength established therein, the formation of the acoustic pressure node takes place in an intermediate position between the reflector wall and the interface (120), approximately in a position corresponding to 1/3-2/3 of channel (110) width, respectively. The particles (101) will tend to become concentrated in the nodal position of the standing wave from the moment, during their circulation along the channel, that they enter the active zone of the acoustic field.

The rest of the suspension components (150) are not affected by the acoustic field, will not cross the interface (120) between the two media (150 and 130) and continue circulating, flowing along their corresponding bed (122) throughout the micro-fluidic channel (110), until reaching the branching point (175) as of which they will abandon the device through the outflow channel-branch (170).

Worth mentioning is the high degree of effectiveness of the selective separation and extraction of the large 20 µm particles obtained during the experiments with the device of the invention.

For example, in experiments where the samples were injected into the treatment channel using 5 ml syringes at a circulation speed of 1.4 mm/s (12 minutes for emptying 1 ml) in the channel, no negative action results were found wherein large 20 µm particles abandoned the device through the suspension outflow channel, but rather were continuously transported towards the collector fluid, through the outflow channel of which they were discharged.

A qualitative analysis of visualization of the samples collected at the exit of the two channels (170) and (180) confirms the effectiveness of the selective separation and extraction of the 20 µm particles of the suspension wherein they were immersed prior to ultrasonic treatment. The liquid collected for one minute from the channel wherethrough the suspension subjected to the acoustic wave is discharged does not contain 20 µm particles but, however, reveals a very high presence of smaller particles, with diameters of 6 µm. On the contrary, the liquid collected at the exit of the channel (180) contains 20 µm polystyrene particles which, as can be previously observed in the central channel (110) and in the branching zone (175), are acoustically separated from their initial suspension and extracted to the collector fluid (124), abandoning the device through the channel (180).

These experiments were conducted at a very low concentration for 20 µm particles, far below 1% of their volumetric concentration, simulating real situations of tumour cells exfoliated from blood. The repetitive behavior found in this particle population at different concentrations (always less than 10%) is due to and understood as the individual action of the acoustic radiation force exerted upon each particle, regardless of its concentration in the suspension. The effectiveness of the action is valid for both high concentrations and extremely diluted suspensions, where other separation techniques show a sharp reduction in sensitivity and effectiveness.

## Claims

1. A micro-device for selective and non-invasive separation and extraction of particles in polydispersed suspensions, **characterized in that** it comprises the following components, integrated in a chip substrate of acoustically soft material:
a) a flow microchannel system having an asymmetrical spatial distribution of the outflow channelling branches stemming from the central treatment channel (110) which comprises (see Figure 1):
i. a path or bed wherealong the starting suspension flows, which includes an inflow channel (160) for supply and an outflow channel (170) wherethrough it abandons the device, in parallel with
ii. a path or bed wherealong the pure fluid (124) wherefrom the selected particles will be extracted (called a collector fluid bed) flows, which includes an inflow or supply channel (162) and an outflow channel (180) that form
iii. a central treatment channel (110) where the starting suspension and pure fluid are separated by a border interface of streamlines under laminar flow regime defined by the transverse dimensions of the channel, which branches off at the end of its path (175) into two outflow channels (170, 180), where the channel (110) width is substantially smaller than a quarter of the acoustic wavelength, which forms its cross-section,
and
b) an ultrasonic actuator or transducer on one of the side walls (190)
- external and parallel to the central treatment channel (110), which transmits acoustic energy to the chip-channel assembly forming a multi-layer system, the resonance of which allows the pressure node to be correctly positioned inside the channel at a distance of approximately 1/3 and 2/3 of channel (110) width, respectively, from the reflector wall, the scope of action of which includes the treatment channel (110), in addition to the branching zone towards the two outflow microchannels, with the object of maximising selective separation and extraction efficiency.

2. A micro-device, according to claim 1, **characterized in that** channel (110) width has a dimension slightly larger than a quarter of the acoustic wavelength.

3. A micro-device, according to claim 1, **characterized in that** channel (110) depth has a dimension considerably smaller than a quarter of the acoustic wavelength.

4. A micro-device, according to claim 1, **characterized in that** the acoustically soft material of the chip substrate is a material with an impedance that does not exceed three times the impedance of the liquid media, within a range of variability that varies between 0.8 MRayls and 2.6 MRayls.

5. A micro-device, according to claim 4, **characterized in that** the acoustically soft material of the chip substrate is an acrylic material.

6. A micro-device, according to claim 1, **characterized in that** the acrylic material is an epoxy resin SU-8.

7. A micro-device, according to claim 1, where the piezoelectric transducer is a piezoelectric ceramic or piezoelectric composite.

8. A micro-device, according to claim 7, **characterized in that** the piezoelectric composite is of 1-3 class.

9. A process for manufacturing the micro-device described in claims 1 to 8 using the hot-stamping technique, **characterized in that** it comprises the following stages:
a) Deposition and definition of a photodefinable polymer layer on the surface of an independent substrate (wafer 1),
b) Deposition and definition of a photodefinable polymer SU-8 layer on the surface of an independent substrate (wafer 2) covered by a non-stick material,
c) Sealing of wafer 1 and wafer 2, disposing said wafers in opposition to each other on the side containing the photodefinable polymer material, and
d) Removal of the wafer covered by non-stick material.

10. A process for manufacturing the micro-device described in claims 1 to 8 using the hot-stamping technique combined with a subsequent gluing process, which comprises the following stages:
a) Preparation of a mould wherein the desired channel designs are included,
b) Molding of the substrate to be used using the mould obtained in a) under the action of pressure and/or temperature, and
c) Sealing of the substrate by gluing to another plastic material under the action of pressure and/or temperature and/or oxygen plasma surface activation.

11. Use of the micro-device claimed in claims 1 to 10 in a process for the selective and non-invasive separation, washing and/or classification of particles in polydispersed suspensions.

12. Use, according to claim 11, **characterized in that** the particles are selected from among:
- virus,
- prions, and
- cells.

13. Use, according to claim 12, **characterized in that** the cells are prokaryotic cells.

14. Use, according to claim 13, **characterized in that** the prokaryotic cells are bacteria.

15. Use, according to claim 13, **characterized in that** the cells are eukaryotic cells.

16. Use, according to claim 15, **characterized in that** the eukaryotic cells are selected from among:
- fungi,
- algae, and
- human cells.

17. Use, according to claim 16, **characterized in that** the human cells are selected from among:
- tumour cells,
- blood cells,
- stem cells, and
- parent cells.

18. Use, according to claim 17, **characterized in that** the parent cells are somatic.

19. Use, according to claim 17, **characterized in that** the parent cells are embryonic.

20. Use, according to claim 16, **characterized in that** the fungi are yeasts.

21. Use of the micro-device claimed in claims 1 to 10, **characterized in that** the eukaryotic cell separation process is carried out in blood, plasmapheresis processes, dialysis processes and laboratory analysis, as well as in blood recycling and/or washing processes after surgical operations.

22. Use of the micro-device claimed in claims 1 to 10, **characterized in that** the eukaryotic cell separation process has the object of selective separation and extraction of circulating tumour cells (CTC) in the peripheral blood of oncology patients for the diagnosis and prognosis of cancer in human beings.

23. Use, according to the preceding claim, **characterized in that** selective separation and extraction is carried out on patient cells, which may be repaired ex vivo and re-administered to the patient.

24. Use, according to claim 22, in the isolation of human or animal parent cells from different tissues or fluids.
